# EUROPEAN PATENT SPECIFICATION

(11) **EP 4 076 545 B1**
(45) Date of publication and mention of the grant of the patent: **30.04.2025**
(21) Application number: 20841686.7
(22) Date of filing: 18.12.2020
(51) Int. Cl.: A61L 9/12

(54) **DEVICE FOR DIFFUSING VOLATILE SUBSTANCES**
VORRICHTUNG ZUM ZERSTREUEN VON FLÜCHTIGEN SUBSTANZEN
DISPOSITIF DE DIFFUSION DE SUBSTANCES VOLATILES

(30) Priority: 18.12.2019 ES 201931122
(43) Date of publication of application: 26.10.2022
(73) Proprietor: Zobele Holding SpA, 38100 Trento (IT)
(72) Inventor: TRÍAS LAFUENTE, Marina, 08019 BARCELONA (ES); GRAUS FERRER, Alba, 08019 BARCELONA (ES); ALFONSO GALLEGO, Fernando, 08019 BARCELONA (ES)
(74) Representative: Herrero & Asociados, S.L.
(86) International application number: PCT/EP2020/086971
(87) International publication number: WO 2021/123125

(56) References cited:
- WO-A1-2014/114820
- WO-A1-2018/071725
- WO-A1-2021/069579
- US-A- 4 889 286
- US-B1- 9 474 818

## Description

The present invention relates to a device for diffusing volatile substances, in particular to an inverted positioning static air freshener.

### Background of the invention

There are inverted positioning static air freshener on the market. As they are products in an inverted position, the difficulty of the solutions consists in the correct dosage of the formulation, both at constant temperature and in cycles of temperature change. In existing products, this solution consists of an intermediate wick fixed to a container by means of a stop.

By placing the container on its base, this intermediate wick comes into contact with a secondary wick. The intermediate wick functions as a dispenser for the formulation, and the secondary wick as an evaporation surface.

This construction allows that when there is an overdose of the formulation due to an increase in temperature, it is returned to the container automatically when it cools.

A drawback of this type of inverted positioning static air fresheners is that the intermediate wick forms part of the fragrance container, so that good contact between the intermediate wick and the secondary wick cannot be ensured.

Furthermore, another drawback is that dripping may occur at the time of activation, and the product is for single use only.

US9474818B1 discloses an air freshener that includes a base and a reservoir. The base includes a fan housed therein and one or more base openings extending through a sidewall of the base. The one or more base openings comprise an inlet opening and an outlet opening. The base also includes a coupling receiver positioned on an upper surface of the base. The reservoir includes a reservoir chamber configured to hold a scented liquid therein and an outlet.

WO2014114820A1 discloses a dispenser applicable to air fresheners comprising a container which contains a perfuming liquid and is closed by means of a wooden stopper. Said wooden stopper contains a central axial duct having a small diameter, which communicates with a second transverse opening arranged in an upper semicylindrical extension, through which the diffusing string or wick is guided.

US4889286A discloses a controllable air freshener unit in which a bottle filled with a volatile liquid fragrance is covered by a cap having vent holes therein that are covered by an absorbent membrane formed of microporous material disposed on the underside of the cap.

WO2018071725A1 discloses a fragrance diffuser has a diffuser element connecting a first reservoir to a second reservoir. As a fragrant oil flows between the reservoirs, the diffuser element becomes saturated with oil and diffuses the fragrance or scent.

WO2021069579 A1, which is prior art according to Article 54(3) EPC discloses a device for diffusing volatile substances comprises a container containing a liquid with said volatile substances, a base element that closes said container, and a wick (3) that is impregnated with said volatile substances, wherein the base element comprises an internal channel in which the liquid containing the volatile substances is deposited, a part of the wick being placed in said internal channel, being impregnated with the liquid containing the volatile substances located in the channel internal.

### Description of the invention

With the device for diffusing volatile substances of the invention, the aforementioned drawbacks are solved, presenting other advantages that will be described below.

The device for diffusing volatile substances according to the present invention is defined in claim 1, and it comprises:
a container that includes a mouthpiece, the container containing a liquid with volatile substances and
a wick that is impregnated with said liquid contained in the container,
and it also comprises a base located around the mouthpiece of the container, the wick being fixed inside said base.

Furthermore, according to the invention, the mouthpiece of the container comprises a cap that closes the mouthpiece provided with a hole, the cap being in contact with the wick.

According to a preferred embodiment, the base is a hollow body provided with at least one side hole.

Preferably, the base is removably fixed to said container, in any suitable way, for example, by means of a thread or by pressure.

Furthermore, the container may comprise a membrane that allows air to escape, but prevents the liquid from escaping.

If desired, the mouthpiece of the container may comprise a dosing pad.

Preferably, the cap is positioned around the outside of the container mouthpiece, for example screwed onto it.

According to a preferred embodiment, the membrane is located at an opposite end of the mouthpiece of the container.

Furthermore, in the position of use of the device for diffusing volatile substances according to the present invention, the mouthpiece of the container is located at the lower end of the container, and the membrane is located at the upper end of the container.

The device for diffusing volatile substances according to the present invention has, among others, the following advantages:
- Avoids leaks;
- Ensures the correct dosage and evaporation of the formulation that is inside the container;
- The wick incorporates a system for fixing and easy releasing the subassembly without the need for the user to come into contact with the impregnated wick.
- Allows the reuse of parts.

### Brief description of the drawings

In order to better understanding what has been stated, some drawings are attached in which, schematically and only as a non-limiting example, a practical case of embodiment is represented.

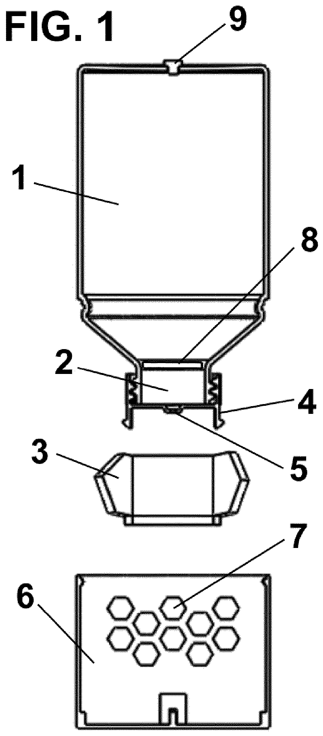

Figure 1 is an elevation section view of the device for diffusing volatile substances according to the present invention.

### Description of a preferred embodiment

As shown in Figure 1, the device for diffusing volatile substances according to the present invention comprises a container 1 inside which a liquid is housed containing the volatile substances that will be diffused into the environment.

Said volatile substances may be used for perfuming the environment, using the device as an air freshener, or against insects, using the device as an insecticide.

The container 1 comprises a mouthpiece 2 through which the liquid comes out, which is impregnated in a wick 3, from which it diffuses into the environment.

Around said mouthpiece 2 a cap 4 is placed that is in contact with the wick 3. If desired, the wick 3 can be joined to the cap 4. The function of the cap 4 is to close the mouthpiece 2, except for a hole 5 of the cap 4, where the liquid comes out to impregnate the wick 3.

Said cap 4 is placed in the mouthpiece 2 in any suitable way, for example, by means of a thread, as shown in figure 1.

The device for diffusing volatile substances according to the present invention also comprises a base 6 provided with a plurality of holes 7.

Said base 6 is preferably a hollow body that is removably connected to the container 1, for example, by means of a thread or pressure, and houses inside the wick 3 and the area of the mouthpiece 2 of the container 1, as can be deduced from figure 1.

Optionally, the container 1 can comprise a dosing pad 8 to control the dosage of the liquid to the wick 3. This dosing pad 8 can be placed close to the mouthpiece 2 of the container 1, although it is optional and not essential.

Furthermore, the container 1 of the diffusion device according to the present invention also comprises a membrane 9, which is permeable to air, but impermeable to liquids.

As can be seen in figure 1, the diffusion device according to the present invention is used in an inverted position, that is, with the mouthpiece 2 of the container 1 at the bottom, the device being supported on a surface by means of base 6.

In its position of use, the membrane 9 is located at the opposite end to the mouthpiece 2 of the container 1, that is to say, in the upper part of the container 1.

The operation of the device for diffusing volatile substances according to the present invention is very simple. Simply the container 1 is placed in an inverted position on the base 6 and, in this position, the liquid comes out through the hole 5 of the cap 4 and impregnates the wick 3. The wick 3 impregnated with the liquid, which is fixed inside the base 6, shall diffuse the volatile substances into the environment, leaving the base 6 through the holes 7 that are located on the side wall of the base 6.

The membrane 9 located in the upper part of the container 1, in the position of use represented in figure 1, allows the air inside the container 1 to escape from it, but prevents the leakage of the liquid, which will only come out of the container 1 through the hole 5 of the cap 4.

In this way, as the device for diffusing volatile substances is subject to changes in temperature, the membrane 9 makes it possible to release pressure and thus avoid overdosing the liquid.

Despite the fact that reference has been made to a specific embodiment of the invention, it is obvious to a person skilled in the art that the device for diffusing volatile substances described is susceptible to numerous variations and modifications, and that all the mentioned details can be substituted by others technically equivalent, without departing from the scope of the invention as defined by the appended claims.

## Claims

1. Device for diffusing volatile substances, comprising:
a container (1) including a mouthpiece (2), the container (1) containing a liquid with volatile substances; and
a wick (3) that is impregnated with said liquid contained in the container (1), wherein it also comprises a base (6) located around the mouthpiece (2) of the container (1), the wick (3) being fixed inside said base (6);
and **characterized in that** the mouthpiece (2) of the container (1) comprises a cap (4) that closes the mouthpiece (2) provided with a hole (5), the cap (4) being in contact with the wick (3).

2. Device for diffusing volatile substances according to claim 1, wherein the base (6) is a hollow body provided with at least one side hole (7).

3. Device for diffusing volatile substances according to claim 1, wherein the base (6) is removably fixed to said container (1).

4. Device for diffusing volatile substances according to claim 1, wherein the container (1) comprises a membrane (9) that allows air to escape, but prevents the liquid from escaping.

5. Device for diffusing volatile substances according to claim 1, wherein the mouthpiece (2) of the container (1) comprises a dosing pad (8).

6. Device for diffusing volatile substances according to claim 1, wherein the cap (4) is placed around the outside of the mouthpiece (2) of the container (1).

7. Device for diffusing volatile substances according to claim 4, wherein the membrane (9) is located at an opposite end of the mouthpiece (2) of the container (1).

8. Device for diffusing volatile substances according to claim 1, wherein, in its position of use, the mouthpiece (2) of the container (1) is positioned at the lower end of the container (1).

9. Device for diffusing volatile substances according to claims 4 or 7, wherein, in its position of use, the membrane (9) is placed at the upper end of the container (1).

## Patentansprüche

1. Vorrichtung zum Verbreiten von flüchtigen Substanzen, umfassend:
einen Behälter (1) mit einem Mundstück (2), wobei der Behälter (1) eine Flüssigkeit mit flüchtigen Substanzen enthält; und
einen Docht (3), der mit der in dem Behälter (1) enthaltenen Flüssigkeit imprägniert ist,
wobei sie auch eine Basis (6) umfasst, die um das Mundstück (2) des Behälters (1) angeordnet ist, wobei der Docht (3) innerhalb der Basis (6) befestigt ist; und
**dadurch gekennzeichnet, dass**
das Mundstück (2) des Behälters (1) eine Kappe (4) umfasst, die das mit einem Loch (5) versehene Mundstück (2) verschließt, wobei die Kappe (4) mit dem Docht (3) in Kontakt steht.

2. Vorrichtung zum Verbreiten von flüchtigen Substanzen nach Anspruch 1, wobei die Basis (6) ein Hohlkörper ist, der mit mindestens einem Seitenloch (7) versehen ist.

3. Vorrichtung zum Verbreiten von flüchtigen Substanzen nach Anspruch 1, wobei die Basis (6) abnehmbar an dem Behälter (1) befestigt ist.

4. Vorrichtung zum Verbreiten von flüchtigen Substanzen nach Anspruch 1, wobei der Behälter (1) eine Membran (9) umfasst, die Luft entweichen lässt, aber verhindert, dass die Flüssigkeit entweicht.

5. Vorrichtung zum Verbreiten von flüchtigen Substanzen nach Anspruch 1, wobei das Mundstück (2) des Behälters (1) ein Dosierpad (8) umfasst.

6. Vorrichtung zum Verbreiten von flüchtigen Substanzen nach Anspruch 1, wobei die Kappe (4) um die Außenseite des Mundstücks (2) des Behälters (1) platziert ist.

7. Vorrichtung zum Verbreiten von flüchtigen Substanzen nach Anspruch 4, wobei sich die Membran (9) an einem gegenüberliegenden Ende des Mundstücks (2) des Behälters (1) befindet.

8. Vorrichtung zum Verbreiten von flüchtigen Substanzen nach Anspruch 1, wobei das Mundstück (2) des Behälters (1) in seiner Gebrauchsposition am unteren Ende des Behälters (1) positioniert ist.

9. Vorrichtung zum Verbreiten von flüchtigen Substanzen nach Anspruch 4 oder 7, wobei die Membran (9) in ihrer Gebrauchsposition am oberen Ende des Behälters (1) platziert ist.

## Revendications

1. Dispositif pour la diffusion de substances volatiles, comprenant :
un récipient (1) comportant une embouchure (2), le récipient (1) contenant un liquide avec des substances volatiles ; et
une mèche (3) qui est imprégnée dudit liquide contenu dans le récipient (1),
dans lequel il comprend également une base (6) située autour de l'embouchure (2) du récipient (1), la mèche (3) étant fixée à l'intérieur de ladite base (6) ;
et **caractérisé**
**en ce que** l'embouchure (2) du récipient (1) comprend un capuchon (4) qui ferme l'embouchure (2) dotée d'un trou (5), le capuchon (4) étant en contact avec la mèche (3).

2. Dispositif pour la diffusion de substances volatiles selon la revendication 1, dans lequel la base (6) est un corps creux doté d'au moins un trou latéral (7).

3. Dispositif pour la diffusion de substances volatiles selon la revendication 1, dans lequel la base (6) est fixée de manière amovible audit récipient (1).

4. Dispositif pour la diffusion de substances volatiles selon la revendication 1, dans lequel le récipient (1) comprend une membrane (9) qui permet à l'air de s'échapper, mais empêche le liquide de s'échapper.

5. Dispositif pour la diffusion de substances volatiles selon la revendication 1, dans lequel l'embouchure (2) du récipient (1) comprend un tampon de dosage (8).

6. Dispositif pour la diffusion de substances volatiles selon la revendication 1, dans lequel le capuchon (4) est placé autour de l'extérieur de l'embouchure (2) du récipient (1).

7. Dispositif pour la diffusion de substances volatiles selon la revendication 4, dans lequel la membrane (9) est située à une extrémité opposée de l'embouchure (2) du récipient (1).

8. Dispositif pour la diffusion de substances volatiles selon la revendication 1, dans lequel, dans sa position d'utilisation, l'embouchure (2) du récipient (1) est positionnée à l'extrémité inférieure du récipient (1).

9. Dispositif pour la diffusion de substances volatiles selon les revendications 4 ou 7, dans lequel, dans sa position d'utilisation, la membrane (9) est placée à l'extrémité supérieure du récipient (1).
